# EUROPEAN PATENT APPLICATION

(11) **EP 1 334 690 A1**
(43) Date of publication of application: **13.08.2003**
(21) Application number: 01980897.1
(22) Date of filing: 25.10.2001
(51) Int. Cl.: A61B 1/00

(54) **ENDOSCOPE AND MEDICAL DEVICE SUCH AS ENDOSCOPE USED TOGETHER WITH MRI**

(30) Priority: 25.10.2000 JP 2000325065; 25.10.2000 JP 2000325117
(71) Applicant: National Institute of Advanced Industrial Science and Technology, Tokyo 100-8921 (JP); Chinzei, Kiyoyuki, Tukuba-shi, Ibaraki 305-0051 (JP)
(72) Inventor: CHINZEI, Kiyoyuki, Tukuba-shi, Ibaraki 305-0051 (JP)
(74) Representative: Panten, Kirsten
(86) International application number: JP0109355
(87) International publication number: WO02034125

(57) **Abstract**

If the material having low magnetic susceptibility is electrically conductive, the varying magnetic field of MRI and/or the motion of the substance in the magnetic field generates eddy current according to the electromagnetic induction law, and further the induction magnetic field caused by this eddy current changes the magnetic field. The above problems can be solved if the material of a medical instrument such as endoscope satisfies the following conditions: (1) of constituent elements of the medical instrument to be used with MRI, at least the constituent elements to be inserted inside the human body and at least the constituent elements which may contact the human body surface are required to have at least the surface layer and the region electrically conductive to the surface layer via conductive solution, respectively made of insulating material; (2) of constituent elements of the medical instrument to be used with MRI, at least the constituent elements to be placed in the imaging region of MRI and its nearby area are made of insulating material; and (3) the other constituent elements of the medical instrument are made of material (conductive material) having low electric resistance.

## Description

### BACKGROUND OF THE INVENTION

### A) FIELD OF THE INVENTION

The present invention relates to medical instruments such as endoscope, and more particularly to endoscope used in the medical environment such as electric cautery are in use, or inside the imaging region of magnetic resonance imaging (hereinafter called "MRI") and its nearby area, and utilized for diagnosis and/or treatment-instruments such as endoscope (hereinafter called "medical instruments such as endoscope") used inside imaging region of MRI and its nearby area.

### B) DESCRIPTION OF THE RELATED ART

Surgeries using endoscope are increasing nowadays. A presently used endoscope has a metal cover at the tip of the tube.

If the active electrode of an electric cautery inadvertently contacts the endoscope and the body of the patient or the surgeon contacts the endoscope, the contacted body portion receives the electric shock and may cause burn. There are reports of such accidents.

Although the static electromagnetic phenomena caused by the static magnetic field of MRI can be shielded by using material having low magnetic susceptibility, the influence of the dynamic electromagnetic phenomena caused by the gradient magnetic field of MRI or RF pulses cannot be eliminated by low magnetic susceptibility material. The phenomena of the dynamic electromagnetic field include the induction current (eddy current) generated in conductor by the varying magnetic field according to the Faraday's law; the induction current caused by RF pulse in conductor through capacitive coupling; and the attenuation of the resonance signal from the patient body due to the induction of the resonance signal to conductor. The induction current generates an induction magnetic field which distorts the static and dynamic magnetic field of MRI, which may distort the image and lower the signal/noise (S/N) ratio. The attenuation of the resonance signal forms black area in the image.

As of the safety issue, the induction current in a conductor generates heat. If this occurs in the body of the patient, burn may cause to the patient. If the induction current leaks, an electric shock may occur. There are reports of fatal accidents in the U.S.A. during MRI inspection, which accident may be ascribed to burn or electric shock caused by an aneurysm treatment clip made of titanium . Diagnosis using MRI, diagnosis and/or treatment using a combination of MRI and other medical instruments are increasing nowadays. It is necessary that the presence and/or the operation of other medical instruments do not cause artifacts (noises, ghosts , etc.) to MRI, or that the other medical instruments show no malfunction to be caused by MRI.

When MRI is used with other medical instruments, the following conditions are required to be satisfied:
(1) Safety, namely, (a) attraction by magnetic force, (b) heat generation by the induction current, and (c) leakage of the induction current do not exist.
(2) The magnetic field of imaging region and/or its nearby area is not disturbed.
(3) Electric noise affecting the detection of the resonance signal is not generated.
(4) Resonance signals are not induced and attenuated.
(5) The medical instrument is not influenced by the magnetic field of MRI.
(6) The medical instrument is not influenced by the RF pulses of MRI.

Conventionally, material having low magnetic susceptibility is used for endoscope etc. by taking into consideration of the conditions (1)(a), (2) and (5). For such techniques, refer to (1) JP-A-10-3050014 (Endoscope With Low Magnetic Susceptibility), (2) JP-A-10-211184 (Medical Instruments, and Medical Instruments Used With Magneto Resonance Imaging), (3) JP-A-7-303625 (Instrument For Use With Magneto Resonance Imager", (4) Chinzei "Surgery Support and MR-Compatibility, BME (Journal of The ME Society of Japan), (5) Schenck, J. F. : The role of magnetic susceptibility in magnetic resonance imaging : MRI magnetic compatibility of the first and second kinds. Medical Physics, 23, (6) (1996) 815 - 50, (6) Chinzei : MR Compatibility of Mechatronic Devices : Design Criteria. MICCAI'99, (1999), and (7) Chinzei "Open MRI and Robot", Journal of The Robot Society of Japan (18) (1) (2000), 37 - 40.

The inventions described in the Publications of the conditions (1) to (3) are all related to alleviating the magnetic influence of MRI, and do not contain a countermeasure against the dynamic electromagnetic phenomena such as the induction current. Although the publication, particularly of the condition (3), describes the invention pertaining a general countermeasure against electromagnetic phenomena, the influence of the induction current cannot be eliminated by this invention only, and if a surgical instrument is used with MRI, the image will be adversely affected. The present invention solves the disadvantage of the invention of the condition (3).

The papers of the conditions (4) and (5) explain the compatibility of MRI with other instruments, and describe the generation of the induction current and the influence thereof upon the image. However, these papers present only the general explanation and do not describe a specific technique.

The papers of the conditions (6) and (7) describe design examples for a robot which is used at distant from the MRI scanner by 1 m. Since the robot is positioned away from the scanner, the robot is not relevant to the present invention which provides the apparatus to be operated in the imaging region or its nearby area.

As described above, although conventional countermeasures for endoscopes and other medical instruments are effective for the static magnetic field of MRI, they do not sufficiently consider the countermeasure against phenomena to be caused by the induction current. During MRI scanning, dynamically varying electromagnetic field environment is formed so that even it material having low magnetic susceptibility is used for an endoscope or the like, it is not sufficient.

More specifically, if the material having low magnetic susceptibility is electrically conductive, change in the magnetic field of MRI or motion of the substance in the magnetic field generates eddy current according to the law of electromagnetic induction, and further the induction magnetic field caused by this eddy current changes the magnetic field (contradicts the condition (2)).

Also a conductor can be a cause of accidents of heat generation or current leakage of induction current or RF pulses (contradiction to the conditions (1)(b) and (1)(c)). For example, in the fatal accident during MRI inspection, it is said that an aneurysm treatment clip made of titanium has a possibility of the burn or leakage current, and MRI inspection is prohibited to the patients with the clip being buried. This clip may be also associated with the error of the magnetic field to be caused by the induction magnetic field by eddy current (contradiction to the condition (2)) and with the attenuation of a resonance signal dissipating to the clip by its electric resistance (contradiction to the condition (4)).

### SUMMARY OF THE INVENTION

The above problems can be solved if the material of a hard endoscope and other diagnosis and treatment instruments satisfies the following conditions:
(1) At least the surface layer of the tube tip of the endoscope to be inserted inside a human body is made of insulating material.
(2) Constituent elements of the endoscope to be placed in the imaging region of MRI and its nearby area are made of insulating material.
(3) Constituent elements of the endoscope to be placed outside the imaging region of MRI and its nearby area are made of material having a low resistance.
(4) Of constituent elements of a medical instrument such as an endoscope to be used with MRI, at least the constituent elements to be inserted inside the human body and at least the constituent elements which may contact the human body surface are required to have at least the surface layer, and the region electrically conductive to the surface layer via conductive solution, respectively made of insulating material.
(5) Of constituent elements of a medical instrument such as an endoscope to be used with MRI, at least the constituent elements to be placed in the imaging region of MRI and its nearby area are made of insulating material.
(6) Of constituent elements of a medical instrument such as an endoscope, constituent elements other than those described in (4) and (5) are made of material (conductive material) having low resistance.

The influence of the dynamic electromagnetic field becomes significant inside a conductive material and its nearby area (about several centimeters). This influence can be eliminated by using insulating material as the material of constituent elements of a medical instrument such as an endoscope to be placed in the imaging region of MRI and its nearby area.

The safety issue occurs at least at constituents elements to be inserted inside the human body and at least constituent elements which may contact the human body surface. Therefore, these constituent elements of a diagnosis or treatment instrument are required to have at least the surface layer and the region electrically conductive to the surface layer via conductive solution, respectively made of insulating material.

The other constituent elements of a medical instrument such as an endoscope may be made of conductive material if necessary, because the influence upon image of MRI is less significant and there is no safety issue.

### BRIEF DESCRIPTION OF THE DRAWINGS

Fig. 1 is a diagram showing the outline of an endoscope according to the invention.
Fig. 2 is a diagram partially in a cross section of the endoscope which is inserted inside a human body, according to the invention.

### DESCRIPTION OF THE PREFERRED EMBODIMENT

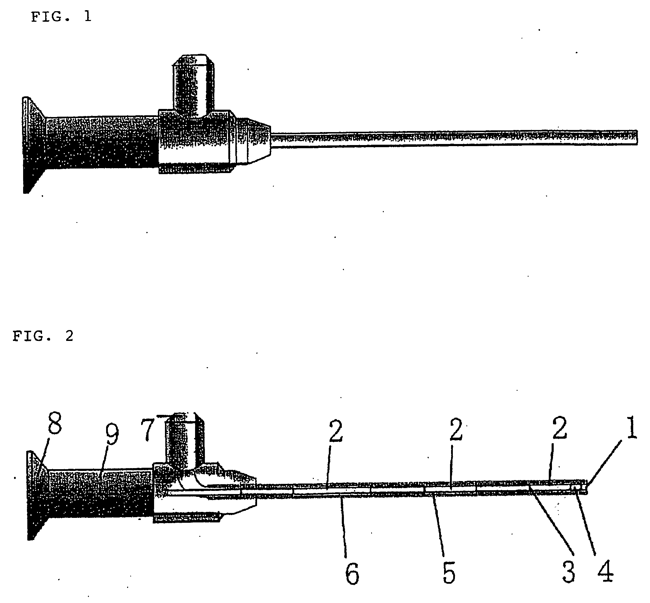

Fig. 1 shows an endoscope according to an embodiment of the invention, and Fig. 2 shows a partial cross section of the endoscope to be inserted inside a human body. The constituent elements to be inserted inside a human body include at least one optical system comprising an objective lens 1 and one or several relay lenses 2 downstream of the objective lens 1. Spacers 3 and a void region 4 are inserted between the objective lens 1 and the relay lens 2 and between the relay lenses 2, at positions where and when necessary. A prism (not shown) may be placed between the objective lens 1 and relay lenses 2 to observe the oblique direction, or the objective lens 1 may be provided with the function of the prism.

Illumination optical fiber is placed in parallel to the optical system. A channel (not shown) may be formed for Hashing water or other material therethrough. The outer surface is covered with a protective cover 6.

Constituent elements of the endoscope not inserted inside the human body includes a connection unit 7 for the illumination optical fiber, an eyepiece unit 8 and an outer envelope 9. The constituent elements from the connection unit 7 to outer envelope 9 may be made of conductive material, e.g., metal material such as brass, aluminum and titanium.

Typical insulating material of the constituent elements from the objective lent 1 to protective cover 6 may be various resin, solid such as silicon dioxide and ceramic, gas such as air, or insulating liquid. In this embodiment, the objective lens I is made of silicon dioxide (SiO₂). The magnetic susceptibility of silicon dioxide is in the order of 10⁻⁵ (system of SI unit). Although this susceptibility is smaller than that of the air in the downstream void region 4, the static electromagnetic field hardly exists and the MRI operation is not influenced.

The advantages of the invention are as follows:
(1) Safety from heat generation by induction current can be ensured.
(2) No leakage current.
(3) No electric noise which influences resonance signal detection.
(4) No induction and attenuation of resonance signals.
(5) RF pulses of MRI do not influence other instruments.
(6) No electric shock particularly of an endoscope.

The present invention has been described in connection with the preferred embodiments. The invention is not limited only to the above embodiments. It is apparent that various modifications, improvements, combinations, and the like can be made by those skilled in the art.

## Claims

1. An endoscope wherein at least the surface layer of a tube tip of the endoscope to be inserted inside human body is made of insulating material.

2. An endoscope wherein constituent elements of the endoscope to be placed in imaging region of MRI and its nearby area are made of insulating material.

3. An endoscope according to claim 1 or 2, wherein constituent elements of the endoscope to be placed outside the imaging region of MRI and its nearby area are made of material having low electric resistance.

4. A medical instrument including endoscope wherein of constituent elements of the medical instrument such as endoscope to be used with MRI, at least the constituent elements to be inserted inside human body and at least the constituent elements which may contact the human body surface are required to have at least the surface layer and the region electrically conductive to the surface layer via conductive solution, respectively made of insulating material.

5. A medical instrument including endoscope wherein of constituent elements of the medical instrument to be used with MRI, at least the constituent elements to be placed in the imaging region of MRI and its nearby area are made of insulating material.

6. A medical instrument including endoscope according to claim 4 or 5, wherein of constituent elements of the medical instrument, constituent elements to be placed outside the imaging region of MRI and its nearby area are made of material having low electric resistance.
